(19) Europäisches Patentamt
European Patent Office
Office européen des brevets

(11) Publication number: **0 345 956 B1**

(12) **EUROPEAN PATENT SPECIFICATION**

(45) Date of publication of patent specification: **16.03.94**

(51) Int. Cl.[5]: **C07D 403/06**, A61K 31/415, C07C 251/00

(21) Application number: **89305083.1**

(22) Date of filing: **19.05.89**

The file contains technical information submitted after the application was filed and not included in this specification

(54) **Tricyclic ketones.**

(30) Priority: **20.05.88 GB 8812002**

(43) Date of publication of application:
**13.12.89 Bulletin 89/50**

(45) Publication of the grant of the patent:
**16.03.94 Bulletin 94/11**

(84) Designated Contracting States:
**AT BE CH DE ES FR GB GR IT LI LU NL SE**

(56) References cited:
**EP-A- 0 191 562**
**EP-A- 0 210 840**
**EP-A- 0 219 193**
**EP-A- 0 242 973**
**EP-A- 0 307 145**
**DE-A- 3 740 352**
**GB-A- 2 153 821**

(73) Proprietor: **GLAXO GROUP LIMITED**
**Clarges House**
**6-12 Clarges Street**
**London W1Y 8DH(GB)**

(72) Inventor: **Price, Barry John**
**5 Cowpers Way**
**Tewin Wood Hertfordshire(GB)**
Inventor: **Coates, Ian Harold**
**20 Mandeville Road**
**Hertford Hertfordshire(GB)**
Inventor: **Bradshaw, John**
**1 Putty Hall Cottages**
**Shaftenhoe End**
**Barley Royston Hertfordshire(GB)**
Inventor: **Mitchell, William Leonard**
**230 Barker Drive**
**London, NW1 0JF(GB)**

(74) Representative: **Marchant, James Ian et al**
**Elkington and Fife**
**Prospect House**
**8 Pembroke Road**
**Sevenoaks, Kent TN13 1XR (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

EP 0 345 956 B1

## Description

This invention relates to tricyclic ketones, to processes for their preparation, to pharmaceutical compositions containing them and to their medical use.

In particular the invention relates to compounds which are potent and selective antagonists of 5-hydroxytryptamine (5-HT) at 5-HT receptors of the type located on terminals of primary afferent nerves. Receptors of this type are now designated as 5-$HT_3$ receptors and are also present in the central nervous system. 5-HT occurs widely in the neuronal pathways in the central nervous system and disturbance of these 5-HT containing pathways is known to alter behavioural syndromes such as mood, psychomotor activity, appetite and memory.

Compounds having antagonist activity at 5-$HT_3$ receptors have been described previously.

Thus for example published UK Patent Specification No. 2153821A and published European Patent Specifications Nos. 191562, 219193 and 210840 disclose 3-imidazolylmethyltetrahydrocarbazolones which may be represented by the general formula (A):

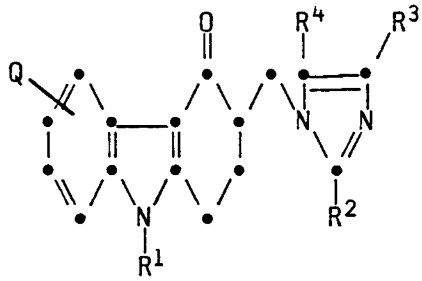

(A)

wherein $R^1$ represents a hydrogen atom or a group selected from $C_{1-10}$alkyl, $C_{3-6}$alkenyl, $C_{3-10}$alkynyl, $C_{3-7}$cycloalkyl, $C_{3-7}$cycloalkyl$C_{1-4}$alkyl, phenyl or phenyl$C_{1-3}$alkyl, and in the case where Q represents a hydrogen atom, $R^1$ may also represent $-CO_2R^5$,$-COR^5$, $-ONR^5R^6$ or $-SO_2R^5$ (wherein $R^5$ and $R^6$, which may be the same or different, each represents a hydrogen atom, a $C_{1-6}$alkyl or $C_{3-7}$cycloalkyl group, or a phenyl or phenyl$C_{1-4}$alkyl group, in which the phenyl group is optionally substituted by one or more $C_{1-4}$alkyl, $C_{1-4}$alkoxy or hydroxy groups or halogen atoms, with the proviso that $R^5$ does not represent a hydrogen atom when $R^1$ represents a group $-CO_2R^5$ or $-SO_2R^5$);
one of the groups represented by $R^2$, $R^3$ and $R^4$ is a hydrogen atom or a $C_{1-6}$alkyl, $C_{3-7}$cycloalkyl, $C_{2-6}$alkenyl, or phenyl$C_{1-3}$alkyl group, and each of the other two groups, which may be the same or different, represents a hydrogen atom or a $C_{1-6}$alkyl group;
Q represents a hydrogen atom or a halogen atom or a hydroxy, $C_{1-4}$alkoxy, phenyl$C_{1-3}$alkoxy or $C_{1-6}$alkyl group or a group $-NR^7R^8$ or $-CONR^7R^8$ (wherein $R^7$ and $R^8$, which may be the same or different, each represents a hydrogen atom or a $C_{1-4}$alkyl or $C_{3-4}$alkenyl group, or together with the nitrogen atom to which they are attached form a saturated 5 to 7 membered ring);
and physiologically acceptable salts and solvates thereof.

Furthermore, published European Patent Specification No. 242973 discloses ketone derivatives which may be represented by the general formula (B):

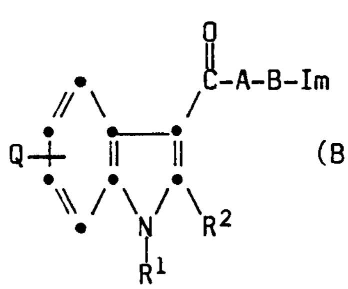

(B)

wherein Im represents an imidazolyl group of formula:

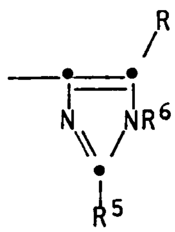

or

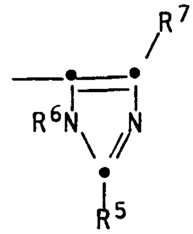

Q represents a hydrogen atom;
$R^1$ represents a hydrogen atom or a $C_{1-6}$alkyl, $C_{3-6}$alkenyl, $C_{3-10}$alkynyl, $C_{3-7}$cycloalkyl, $C_{3-7}$cycloalkyl$C_{1-4}$alkyl, phenyl or phenyl$C_{1-3}$alkyl group;
$R_2$ represents a hydrogen atom or a $C_{1-6}$alkyl, $C_{3-6}$alkenyl, $C_{3-7}$cycloalkyl, phenyl or phenyl$C_{1-3}$alkyl group;
A-B represents the group $R^3R^4C\text{-}CH_2$ or $R^3C=CH$;
$R^3$ and $R^4$, which may be the same or different, each represents a hydrogen atom or a $C_{1-6}$alkyl group;
one of the groups represented by $R^5$,$R^6$ and $R^7$, is a hydrogen atom or a $C_{1-6}$alkyl, $C_{3-7}$cycloalkyl, $C_{3-6}$alkenyl, phenyl or phenyl$C_{1-3}$alkyl group, and each of the other two groups, which may be the same or different, represents a hydrogen atom or a $C_{1-6}$alkyl group;
and physiologically acceptable salts and solvates thereof.

Published European Patent Specification No. 266899 discloses ketone derivatives which may also be represented by the general formula (B), but wherein Im represents an imidazolyl group of formula:

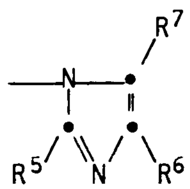

wherein $R^5$, $R^6$ and $R^7$ are as defined in EPA 242973, although one of these groups may additionally represent a vinyl group;
$R^1$ is as defined in EPA 242973, although one of these groups may additionally represent a group selected from $-CO_2R^{10}$, $-COR^{10}$, $-CONR^{10}R^{11}$ or $-SO_2R^{10}$ (wherein $R^{10}$ and $R^{11}$ which may be the same or different, each represents a hydrogen atom, a $C_{1-6}$alkyl or $C_{3-7}$cycloalkyl group, or a phenyl or phenyl$C_{1-4}$alkyl group, in which the phenyl group is optionally substituted by one or more $C_{1-4}$alkyl, $C_{1-4}$alkoxy or hydroxy groups or halogen atoms, with the proviso that $R^{10}$ does not represent a hydrogen atom when $R^1$ represents a group $-CO_2R^{10}$ or $SO_2R^{10}$);
$R^2$ is as defined in EPA 242973;
A-B represents the group $R^3R^4C\text{-}CH_2$ wherein $R^3$ and $R^4$ are as defined in EPA 242973;
Q is as defined in formula (A);
and physiologically acceptable salts and solvates thereof.

British Patent Specification No.2202530A, which was unpublished at the priority date of the present application, discloses compounds of the general formula:

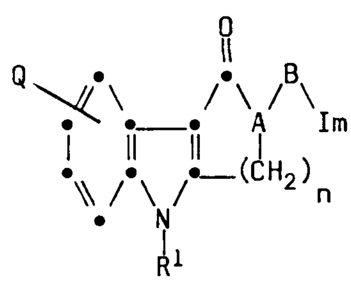

wherein Im represents an imidazolyl group of the formula:

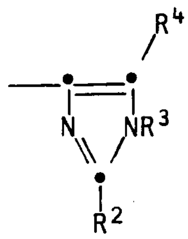

or

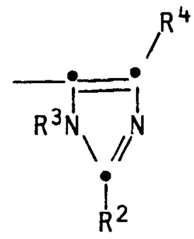

$R^1$ represents a hydrogen atom or a group selected from $C_{1-6}$alkyl, $C_{3-6}$alkenyl, $C_{3-6}$alkynyl, $C_{3-7}$cycloalkyl, $C_{3-7}$cycloalkyl$C_{1-4}$alkyl, phenyl, phenyl$C_{1-3}$alkyl, $-CO_2R^5$, $-COR^5$, $-CONR^5R^6$ or $-SO_2R^5$ - (wherein $R^5$ and $R^6$, which may be the same or different, each represents a hydrogen atom, a $C_{1-6}$alkyl or $C_{3-7}$cycloalkyl group, or a phenyl or phenyl$C_{1-4}$alkyl group, in which the phenyl group is optionally substituted by one or more $C_{1-4}$alkyl, $C_{1-4}$alkoxy or hydroxy groups or halogen atoms, with the proviso that $R^5$ does not represent a hydrogen atom when $R^1$ represents a group $-CO_2R^5$ or $-SO_2R^5$);
one of the groups represented by $R^2$, $R^3$ and $R^4$ is a hydrogen atom or a $C_{1-6}$alkyl, $C_{3-7}$cycloalkyl, $C_{3-6}$alkenyl, phenyl or phenyl$C_{1-3}$alkyl group, and each of the other two groups, which may be the same or different, represents a hydrogen atom or a $C_{1-6}$alkyl group;
Q is as defined in formula (A);
n represents 1, 2 or 3;
and A-B represents the group $CH-CH_2$ or $C=CH$; and physiologically acceptable salts and solvates thereof.

We have now found a novel group of compounds, which although generically covered by the compound definition in GB2202530A, are not specifically disclosed therein, and which are also potent antagonists of the effect of 5-HT at 5-$HT_3$ receptors. Furthermore the compounds of the present invention have a surprisingly advantageous duration of action.

Thus, the present invention provides a tricyclic ketone of the general formula (I):

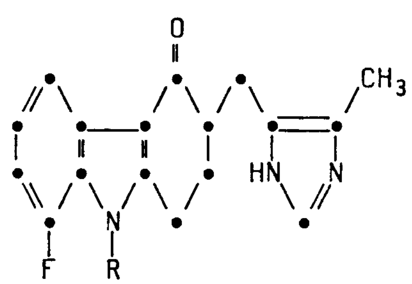

(I)

wherein R represents a hydrogen atom or a $C_{1-3}$alkyl (e.g. methyl) group; and physiologically acceptable salts and solvates thereof.

Suitable physiologically acceptable salts of the compounds of general formula (I) include acid addition salts formed with organic or inorganic acids for example, hydrochlorides, hydrobromides, sulphates, alkyl- or arylsulphonates (e.g. methanesulphonates or p-toluenesulphonates), phosphates, acetates, citrates, succinates, tartrates, fumarates and maleates. The solvates may, for example, be hydrates.

It will be appreciated that the carbon atom at the 3-position of the tetrahydrocarbazolone ring is asymmetric and may exist in the R-or S- configuration. All optical isomers of compounds of general formula (I) and their mixtures including the racemic mixtures thereof, are embraced by the invention.

Referring to the general formula (I), the alkyl group represented by R may be a straight chain or branched chain alkyl group, for example, methyl, ethyl, propyl or prop-2-yl, most preferably methyl.

A particularly preferred compound according to the invention is:
8-fluoro-1,2,3,9-tetrahydro-9-methyl-3-[(5-methyl-1H-imidazol-4-yl)methyl]-4H-carbazol-4-one and its physiologically acceptable salts and solvates.

A further preferred compound according to the invention is:
8-fluoro-1,2,3,9-tetrahydro-3[(5-methyl-1H-imidazol-4-yl)methyl]-4H-carbazol-4-one and its physiologically acceptable salts and solvates.

The potent and selective antagonism of 5-HT at 5-$HT_3$ receptors by compounds of the invention has been demonstrated by their ability to inhibit 3-(5-methyl-1H-imidazol-4-yl)-1-[1-(methyl-$t_3$)-1H-indol-3-yl]-1-propanone binding in rat entorhinal cortex homogenates (following the general procedure described by G. Kilpatrick et al. in Nature, 1987, 330, 746), and/or by their ability to inhibit the 5-HT-induced depolarisation of the rat isolated vagus nerve preparation.

Compounds of formula (I), which antagonise the effect of 5-HT at 5-$HT_3$ receptors, are useful in the treatment of conditions such as psychotic disorders (e.g. schizophrenia and mania); anxiety; and nausea and vomiting, particularly that associated with cancer chemotherapy and radiotherapy. Compounds of formula (I) are also useful in the treatment of gastric stasis; symptoms of gastrointestinal dysfunction such as occur with dyspepsia, peptic ulcer, reflux oesophagitis, flatulence and irritable bowel syndrome; migraine; and pain. Compounds of formula (I) may also be used in the treatment of dependency on drugs and substances of abuse, depression, and dementia and other cognitive disorders.

According to another aspect, the invention provides a method of treatment of a human or animal subject suffering from a psychotic disorder such as schizophrenia or mania; or from anxiety; nausea or vomiting; gastric stasis; symptoms of gastrointestinal dysfunction such as dyspepsia, reflux oesophagitis, peptic ulcer, flatulence and irritable bowel syndrome; migraine; pain; dependency on drugs and substances of abuse; depression; or dementia or another cognitive disorder, which comprises administering an effective amount of a compound of formula (I) or a physiologically acceptable salt or solvate thereof.

Accordingly, the invention also provides a pharmaceutical composition which comprises at least one compound selected from compounds of the general formula (I), and their physiologically acceptable salts and solvates (e.g. hydrates), for use in human or veterinary medicine, and formulated for administration by any convenient route.

Such compositions may be formulated in conventional manner using one or more physiologically acceptable carriers and/or excipients.

Thus the compounds according to the invention may be formulated for oral, buccal, parenteral or rectal administration or in a form suitable for administration by inhalation or insufflation (either through the mouth or nose).

For oral administration, the pharmaceutical compositions may take the form of, for example, tablets or capsules prepared by conventional means with pharmaceutically acceptable excipients such as binding agents (e.g. pregelatinised maize starch, polyvinylpyrrolidone or hydroxylpropyl methylcellulose); fillers (e.g. lactose, microcrystalline cellulose or calcium hydrogen phosphate); lubricants (e.g. magnesium stearate, talc or silica); disintegrants (e.g. potato starch or sodium starch glycollate); or wetting agents (e.g. sodium lauryl sulphate). The tablets may be coated by methods well known in the art. Liquid preparations for oral administration may take the form of, for example, solutions, syrups or suspensions, or they may be presented as a dry product for constitution with water or other suitable vehicle before use. Such liquid preparations may be prepared by conventional means with pharmaceutically acceptable additives such as suspending agents (e.g. sorbitol syrup, cellulose derivatives or hydrogenated edible fats); emulsifying agents (e.g. lecithin or acacia); non-aqueous vehicles (e.g. almond oil, oily esters, ethyl alcohol or fractionated vegetable oils); and preservatives (e.g. methyl or propyl-p-hydroxybenzoates or sorbic acid). The preparations may also contain buffer salts, flavouring, colouring and sweetening agents as appropriate.

Preparations for oral administration may be suitably formulated to give controlled release of the active compound.

For buccal administration the compositions may take the form of tablets or lozenges formulated in conventional manner.

The compounds of the invention may be formulated for parenteral administration by bolus injection or continuous infusion. Formulations for injection may be presented in unit dosage form e.g. in ampoules or in multi-dose containers, with an added preservative. The compositions may take such forms as suspensions, solutions or emulsions in oily or aqueous vehicles, and may contain formulatory agents such as suspending, stabilising and/or dispersing agents. Alternatively, the active ingredient may be in powder form for constitution with a suitable vehicle, e.g. sterile pyrogen-free water, before use.

The compounds of the invention may also be formulated in rectal compositions such as suppositories or retention enemas, e.g. containing conventional suppository bases such as cocoa butter or other glycerides.

In addition to the formulations described previously, the compounds of the invention may also be formulated as depot preparations. Such long acting formulations may be administered by implantation (for example subcutaneously or intramuscularly) or by intramuscular injection. Thus, for example, the compounds of the invention may be formulated with suitable polymeric or hydrophobic materials (for example as an emulsion in an acceptable oil) or ion exchange resins, or as sparingly soluble derivatives, for example, as a sparingly soluble salt.

For administration by inhalation the compounds according to the invention are conveniently delivered in the form of an aerosol spray presentation from pressurised packs or a nebuliser, with the use of a suitable propellant, e.g. dichlorodifluoromethane, trichlorofluoromethane, dichlorotetrafluoroethane, carbon dioxide or other suitable gas. In the case of a pressurised aerosol the dosage unit may be determined by providing a valve to deliver a metered amount. Capsules and cartridges of e.g. gelatin for use in an inhaler or insufflator may be formulated containing a powder mix of a compound of the invention and a suitable powder base such as lactose or starch.

For intranasal administration, the compounds according to the invention may be formulated as solutions for administration via a suitable metered or unit dose device or alternatively as a powder mix with a suitable carrier for administration using a suitable delivery device.

The compounds of formula (I) may also be administered in combination with other therapeutic agents. Thus, for example, in the treatment of gastric stasis, symptoms of gastrointestinal dysfunction and nausea and vomiting, the compounds of formula (I) may be administered in combination with antisecretory agents such as histamine $H_2$-receptor antagonists (e.g. ranitidine, sufotidine, 1-methyl-5-[[3-[3-(1-piperidinylmethyl)-phenoxy]propyl]amino]-1H-1,2,4-triazole-3-methanol, cimetidine, famotidine, nizatidine or roxatidine) or $H^+K^+$ATPase inhibitors (e.g. omeprazole). In the treatment of nausea and vomiting, compounds of formula (I) may also be administered in combination with dexamethasone.

A proposed dose of the compounds of the invention for administration to man (of approximately 70kg body weight) is 0.001 to 100mg, preferably 0.01 to 50g, more preferably 0.1 to 20mg of the active ingredient per unit dose expressed as the weight of free base, which could be administered, for example, 1 to 4 times per day. It will be appreciated that it may be necessary to make routine variations to the dosage, depending on the age and condition of the patient. The dosage will also depend on the route of administration.

According to another aspect of the invention, compounds of general formula (I), and physiologically acceptable salts or solvates thereof, may be prepared by the general methods outlined hereinafter. In the following description, the group R is as defined for compounds of general formula (I) unless otherwise stated.

According to a first general process (A), a compound of formula (I) may be prepared by hydrogenation of a compound of formula (II):

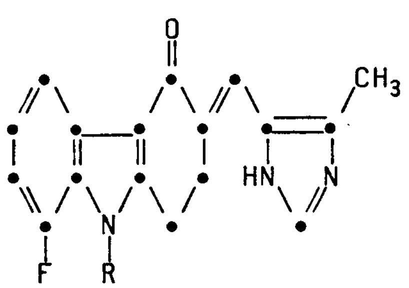

(II)

or a protected derivative thereof, followed where necessary by removal of any protecting groups.

Hydrogenation according to general process (A) may be effected using conventional procedures, for example using hydrogen in the presence of a noble metal catalyst (e.g. palladium, Raney nickel, platinum or rhodium). The catalyst may be supported on, for example, charcoal or alumina, or alternatively a homogeneous catalyst such as tris(triphenylphosphine)rhodium chloride may be used. The hydrogenation will generally be effected in a solvent such as an alcohol (e.g. methanol or ethanol), an ether (e.g. dioxan), a halogenated hydrocarbon (e.g. dichloromethane) or an ester (e.g. ethyl acetate) or mixtures thereof, and at a temperature in the range -20 to +100°C, preferably 0 to 50°C.

Compounds of formula (II) are novel compounds and constitute a further aspect of the invention.

According to another process (B), a compound of formula (I) in which R represents a $C_{1-3}$alkyl group may be prepared by alkylating the compound of formula (I) wherein R represents a hydrogen atom.

The above alkylation reactions may be effected using the appropriate alkylating agent selected from compounds of formula $R^1Z$, wherein $R^1$ represents a $C_{1-3}$alkyl group, and Z represents a leaving atom or group such as a halogen atom (e.g. chlorine, bromine or iodine), an acyloxy group (e.g. trifluoroacetyloxy or acetoxy), or a sulphonyloxy group (e.g. trifluoromethanesulphonyloxy, p-toluenesulphonyloxy or methanesulphonyloxy); or a dialkyl sulphate of formula $(R^1)_2SO_4$.

The alkylation reaction is conveniently carried out in an inert organic solvent such as a substituted amide (e.g. dimethylformamide), an ether (e.g. tetrahydrofuran) or an aromatic hydrocarbon (e.g. toluene), preferably in the presence of a base. Suitable bases include, for example, alkali metal hydrides (e.g. sodium hydride), alkali metal amides (e.g. sodium amide or lithium diisopropylamide), alkali metal carbonates (e.g. sodium carbonate) or an alkali metal alkoxide (e.g. sodium or potassium methoxide, ethoxide or t-butoxide). The reaction may conveniently be effected at a temperature in the range -80 to +100°C, preferably -80 to +50°C.

It should be appreciated that in the above transformations it may be necessary or desirable to protect any sensitive groups in the compound in question to avoid undesirable side reactions. For example, it may be necessary to protect the imidazole nitrogen atom, for example with an arylmethyl (e.g. trityl), alkyl (e.g. t-butyl), alkoxymethyl (e.g. methoxymethyl), acyl (e.g. benzyloxycarbonyl) or a sulphonyl (e.g. N,N-dimethylaminosulphonyl or p-toluenesulphonyl) group.

Thus according to another general process (C), a compound of formula (I) may be prepared by the removal of any protecting groups from a protected form of a compound of formula (I). Deprotection may be effected using conventional techniques such as those described in 'Protective Groups in Organic Synthesis' by T. W. Greene (John Wiley and Sons, 1981).

For example, a trityl group may be cleaved by acid hydrolysis (e.g. using dilute hydrochloric or acetic acid). An alkoxyalkyl group may be removed using a Lewis acid such as boron tribromide. An acyl group may be removed by hydrolysis under acidic or basic conditions (e.g. using hydrogen bromide or sodium hydroxide). A sulphonyl group may be removed by alkaline hydrolysis.

Compounds of formula (II) may be prepared by condensing a compound of formula (III):

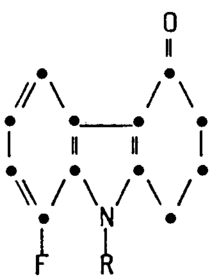

(III)

with the compound of formula (IV):

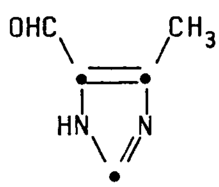

(IV)

or a protected derivative thereof, in the presence of a base such as an alkali metal amide (e.g. lithium diisopropylamide) in an inert solvent such as an ether (e.g. tetrahydrofuran), followed by dehydration. The dehydration process may be effected using conventional methods, for example by using an organic or mineral acid (e.g. p-toluenesulphonic, methanesulphonic, trifluoroacetic or hydrochloric acid) in a suitable solvent such as an ether (e.g. tetrahydrofuran), an alcohol (e.g. methanol), or glacial acetic acid, at a temperature in the range 0 to 100°C.

Compounds of formula (III) may be prepared, for example, by the method or methods analogous to that described by H. Iida et al. in J. Org. Chem., 1980, 45, 2938.

The compound of formula (IV) and protected derivatives thereof may be prepared, for example, by the method described in published European Patent Specification No. 242973A.

Where it is desired to isolate a compound of the invention as a salt, for example a physiologically acceptable salt, this may be achieved by reacting the compound of formula (I) in the form of the free base with an appropriate acid, preferably with an equivalent amount, in a suitable solvent such as an alcohol (e.g. ethanol or methanol), an aqueous alcohol (e.g. aqueous ethanol), an ester (e.g. ethyl acetate) or an ether (e.g. tetrahydrofuran).

Physiologically acceptable salts may also be prepared from other salts, including other physiologically acceptable salts, of the compound of formula (I) using conventional methods.

Individual enantiomers of the compounds of the invention may be obtained by resolution of a mixture of enantiomers (e.g a racemic mixture) using conventional means, such as an optically active resolving acid; see for example 'Stereochemistry of Carbon Compounds' by E.L.Eliel (McGraw Hill, 1962) and 'Tables of Resolving Agents' by S. H. Wilen.

The methods described above for preparing the compounds of the invention may be used for the introduction of the desired groups at any stage in the stepwise formation of the required compounds, and it will be appreciated that these methods can be combined in different ways in such multi-stage processes. The sequence of the reactions in multi-stage processes should of course be chosen so that the reaction conditions used do not affect groups in the molecule which are desired in the final product.

The invention is further illustrated by the following Examples. All temperatures are in °C. Thin layer chromatography (t.l.c.) was carried out on silica, and flash column chromatography (FCC) on silica (Merck 9385). Solvent System A as used for chromatography denotes dichloromethane:ethanol:0.88 ammonia solution. Organic extracts were dried over magnesium sulphate. The following abbreviations are used: THF - tetrahydrofuran; DMF - dimethylformamide.

Intermediate 1

3-[(2-Fluorophenyl)amino]-2-cyclohexen-1-one

2-Fluoroaniline (10g) and cyclohexane-1,3-dione (10g) were heated together under nitrogen at 120° for 1h. The cooled mixture was triturated with ether and filtered to give the title compound (14.8g), m.p. 116-118°.

Intermediate 2

8-Fluoro-1,2,3,9-tetrahydro-4*H*-carbazol-4-one

3-[(2-Fluorophenyl)amino]-2-cyclohexen-1-one (14.8g), palladium (II) acetate (1g), and copper (II) acetate (29.5g) were heated together in DMF (100mℓ) under nitrogen at 140° for 2h. The solvent was removed in vacuo, and the residue was purified by FCC eluting with ether to give the title compound (10.1g), m.p. 222-224°.

Intermediate 3

8-Fluoro-1-methyl-1,2,3,9-tetrahydro-4*H*-carbazol-4-one

To a suspension of sodium hydride (80% dispersion in oil; 1.15g) in dry THF (50mℓ) under nitrogen was added 8-fluoro-1,2,3,9-tetrahydro-4*H*-carbazol-4-one (6.5g) in dry THF (50mℓ), and the mixture was stirred for 1h. Methyl iodide (4.1mℓ) was added, and the mixture was stirred for 3h. The mixture was then poured into brine (300mℓ) and extracted with ether (2x300mℓ). The combined, dried organic extracts were evaporated in vacuo to give the title compound (5.77g), m.p. 126-128°.

Intermediate 4

8-Fluoro-1,2,3,9-tetrahydro-3-[(5-methyl-1*H*-imidazol-4-yl)methylene]-4*H*-carbazol-4-one

Diisopropylamine (3.83mℓ) in dry THF (20mℓ) was cooled to 0° under nitrogen. *n*-Butyllithium (1.69M solution in hexane; 16.3mℓ) was added, and the mixture was stirred for 15 min. before cooling to -78°. 8-fluoro-1,2,3,9-tetrahydro-4H-carbazol-4-one (2.32g) in dry THF (40mℓ) was cooled to -78° under nitrogen, the lithium diisopropylamide solution was added, and the resulting suspension was stirred for 1h. 5-Methyl-1-(triphenylmethyl)-1H-imidazole-4-carboxaldehyde (4.02g) in dry THF (40mℓ) was added, and the mixture was stirred at -78° for 1h, then warmed to 20° and stirred for 3h. Methanesulphonic acid (20mℓ) and acetic acid (30mℓ) were added, and the mixture was heated to 80° for 1h. The cooled mixture was poured into aqueous potassium carbonate solution (500mℓ) and extracted with ethyl acetate (3x50mℓ). The combined, dried organic extracts were evaporated in vacuo to leave an oil which was purified by FCC eluting with System A (75:8:1) to give the title compound (723mg), m.p. 235 - 237° (decomp.).

Intermediate 5

## 8-Fluoro-1,2,3,9-tetrahydro-9-methyl-3-[(5-methyl-1H-imidazol-4-yl)-methylene]-4H-carbazol-4-one

Diisopropylamine (2.35mℓ) in dry THF (l5mℓ) was cooled to 0° under nitrogen. n-Butyllithium (1.24M solution in hexane; 13.4mℓ) was added, and the mixture was stirred for 10 min. before cooling to -78°. 8-fluoro-1-methyl-1,2,3,9-tetrahydro-4H-carbazol-4-one (3g) in dry THF (70mℓ) was cooled to -78° under nitrogen, the lithium diisopropylamide solution was added, and the resulting mixture was warmed to -40° over 1h, before re-cooling to -78°. 5-Methyl-1-(triphenylmethyl)-1H-imidazole-4-carboxaldehyde (4.9g) in dry THF (50mℓ) was added slowly, and the mixture was allowed to warm to 0° over 3h. The mixture was re-cooled to -78°, acetic acid (20mℓ) was added, followed by methanesulphonic acid (50mℓ), and the mixture was warmed to 20° and then heated at reflux for 12h. The cooled mixture was poured into water (400mℓ), basified (to pH10) with potassium carbonate (50g) and extracted with ethyl acetate (3x400mℓ). The combined, dried organic extracts were evaporated in vacuo to give an oil which was purified by FCC eluting with System A (100:8:1) to give the title compound (2.79g), m.p. 260-264° (decomp.).

Example 1

## 8-Fluoro-1,2,3,9-tetrahydro-3-[(5-methyl-1H-imidazol-4-yl)methyl]-4H-carbazol-4-one

8-Fluoro-1,2,3,9-tetrahydro-3-[(5-methyl-1H-imidazol-4-yl)methylene]-4H-carbazol-4-one (728mg) in absolute ethanol (30mℓ) was added to a suspension of 5% palladium on charcoal (100mg) in ethanol (30mℓ), and the resulting mixture was hydrogenated at room temperature and atmospheric pressure until hydrogen uptake had ceased. The mixture was filtered, and the filtrate was evaporated in vacuo to leave an oil which was purified by FCC eluting with ethanol: ethyl acetate (1:5) to give the title compound (342mg), m.p. 135-140°, t.l.c. (ethanol:ethyl acetate, 1:5) Rf 0.175.

Example 2

## 8-Fluoro-1,2,3,9-tetrahydro-9-methyl-3-[(5-methyl-1H-imidazol-4-yl)-methyl]-4H-carbazol-4-one

8-Fluoro-1,2,3,9-tetrahydro-9-methyl-3-[(5-methyl-1H-imidazol-4-yl)methylene]-4H-carbazol-4-one (1.5g) was dissolved in ethanol (50mℓ) and added to a suspension of 5% palladium on charcoal (200mg) in ethanol (ca. 50mℓ), and the resulting mixture was hydrogenated at room temperature and atmospheric pressure until hydrogen uptake had ceased. The mixture was filtered, and the filtrate was evaporated in vacuo to give the title compound (1.5g), m.p. 189-191° (decomp.), t.l.c. (System A 100:8:1), Rf 0.43.

Example 3

**8-Fluoro-1,2,3,9-tetrahydro-3-[(5-methyl-1H-imidazol-4-yl)methyl]-4H-carbazol-4-one maleate**

8-Fluoro-1,2,3,9-tetrahydro-3-[(5-methyl-1H-imidazol-4-yl)methyl]-4H-carbazol-4-one (330mg) was dissolved in dry ethanol (20mℓ). Maleic acid (129mg) in ethanol (10mℓ) was added, and the solution was evaporated in vacuo. The residue was triturated with ether and the resultant solid was crystallised from ethanol:ether to give the title compound (351mg), m.p. 174-177°.

| | | | |
|---|---|---|---|
| Analysis Found: | C,60.6; | H,5.0; | N,9.9; |
| $C_{21}H_{20}FN_3O_5$ requires | C,61.0; | H,4.9; | N,10.2%. |

Example 4

**8-Fluoro-1,2,3,9-tetrahydro-9-methyl-3-[(5-methyl-1H-imidazol-4-yl)-methyl]-4H-carbazol-4-one maleate**

8-Fluoro-1,2,3,9-tetrahydro-9-methyl-3-[(5-methyl-1H-imidazol-4-yl)methyl]-4H-carbazol-4-one (1.51g) was dissolved in ethyl acetate (40mℓ), and added to a solution of maleic acid (563mg) in ethanol (50mℓ). The solution was cooled to 0°, and the resultant solid was filtered off and dried in vacuo to give the title compound (1.61g), m.p. 155-158°, t.l.c. (System A 100:8:1), Rf 0.43.

The following examples illustrate pharmaceutical formulations according to the invention, containing 8-fluoro-1,2,3,9-tetrahydro-9-methyl-3-[(5-ethyl-1H-imidazol-1-yl)methyl]-4H-carbazol-4-one as the active ingredient. Physiologically acceptable salts and/or solvates of this compound, and other compounds of formula (I) and their physiologically acceptable salts and/or solvates may be formulated in a similar manner.

TABLETS FOR ORAL ADMINISTRATION

Tablets may be prepared by the normal methods such as direct compression or wet granulation.

The tablets may be film coated with suitable film forming materials, such as hydroxypropyl methylcellulose, using standard techniques. Alternatively the tablets may be sugar coated.

Direct Compression

| Tablet | mg/tablet |
|---|---|
| Active Ingredient | 0.50 |
| Calcium Hydrogen Phosphate BP* | 87.25 |
| Croscarmellose Sodium NF | 1.80 |
| Magnesium Stearate BP | 0.45 |
| Compression weight | 90.00 |

* of a grade suitable for direct compression.

The active ingredient is passed through a 60 mesh sieve, blended with the calcium hydrogen phosphate, croscarmellose sodium and magnesium stearate. The resultant mix is compressed into tablets using a Manesty F3 tablet machine fitted with 5.5mm, flat bevelled edge punches.

CAPSULES

| | mg/tablet |
|---|---|
| Active Ingredient | 0.5 |
| * Starch 1500 | 98.5 |
| Magnesium Stearate BP | 1.0 |
| Fill Weight | 100.0 |

* a form of directly compressible starch.

The active ingredient is sieved and blended with the excipients. The mix is filled into size No. 2 hard gelatin capsules using suitable machinery. Other doses may be prepared by altering the fill weight an if necessary changing the capsule size to suit.

SYRUP

This may be either a sucrose or sucrose free presentation.

| Sucrose-Free | | mg/5ml dose |
|---|---|---|
| Active Ingredient | | 0.5 |
| Hydroxypropylmethylcellulose USP (viscosity type 4000) | | 22.5 |
| Buffer ) Flavour ) Colour ) Preservative ) Sweetener ) | | as required |
| Purified Water BP | to | 5.0ml |

The hydroxypropylmethylcellulose is dispersed in hot water, cooled and then mixed with an aqueous solution containing the active ingredient and the other components of the formulation. The resultant solution is adjusted to volume and mixed. The syrup is clarified by filtration.

INJECTION FOR INTRAVENOUS ADMINISTRATION

| | mg/mℓ | |
|---|---|---|
| Active ingredient | 0.05 | 0.5 |
| Sodium Chloride BP | as required | as required |
| Water for Injection BP to | 1.0mℓ | 1.0mℓ |

Sodium chloride may be added to adjust the tonicity of the solution and the pH may be adjusted, using acid or alkali, to that of optimum stability and/or facilitate solution of the active ingredient. Alternatively, suitable buffer salts may be used.

The solution is prepared, clarified and filled into appropriate size ampoules sealed by fusion of the glass. The injection is sterilised by heating in an autoclave using one of the acceptable cycles. Alternatively, the solution may be sterilised by filtration and filled into sterile ampoules under aseptic conditions. The solution may be packed under an inert atmosphere of nitrogen or other suitable gas.

## Claims

### Claims for the following Contracting States : AT, BE, CH, DE, FR, GB, IT, LI, LU, NL, SE

**1.** Compounds of the general formula (I)

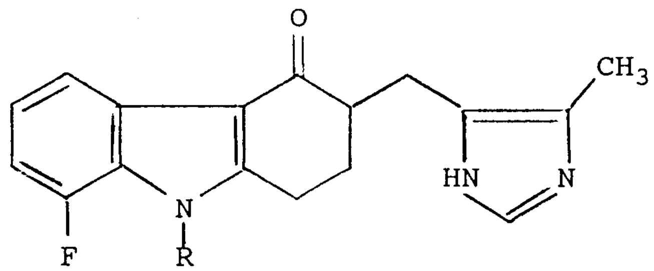

(I)

wherein R represents a hydrogen atom or a $C_{1-3}$alkyl group and physiologically acceptable salts and solvates thereof.

**2.** A compound as claimed in claim 1 characterised in that it is 8-fluoro-1,2,3,9-tetrahydro-9-methyl-3-[(5-methyl-1H-imidazol-4-yl)methyl]-4H-carbazol-4-one and physiologically acceptable salts and solvates thereof.

**3.** A compound as claimed in claim 1 characterised in that it is 8-fluoro-1,2,3,9-tetrahydro-3-[(5-methyl-1H-imidazol-4-yl)methyl]-4H-carbazol-4-one and physiologically acceptable salts and solvates thereof.

**4.** A process for the preparation of compounds as claimed in any of claims 1 to 3 and physiologically acceptable salts and solvates thereof which comprises:

(A) hydrogenating a compound of formula (II)

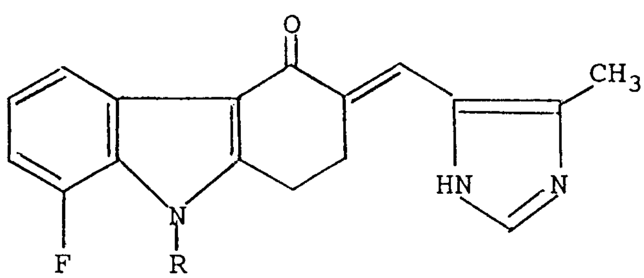

(II)

or a protected derivative thereof, followed where necessary by removal of any protecting group(s) present; or

(B) for the preparation of a compound of formula (I) in which R represents a $C_{1-3}$alkyl group, alkylating the compound of formula (I) in which R represents a hydrogen atom; or

(C) removing one or more protecting groups from a protected form of a compound of formula (I);

and when the compound of formula (I) is obtained as a mixture of enantiomers, optionally resolving the mixture to obtain the desired enantiomer; and/or

where the compound of formula (I) is in the form of a free base, optionally converting the free base into a salt.

**5.** A pharmaceutical composition comprising a compound, of general formula (I) as defined in any of Claims 1 to 3 or a physiologically acceptable salt or solvate thereof together with at least one physiologically acceptable carrier or excipient.

**6.** A compound of general formula (I) as defined in any of Claims 1 to 3 or a physiologically acceptable salt or solvate thereof for use as an active therapeutic agent.

**Claims for the following Contracting States : ES, GR**

**1.** A process for the preparation of compounds of the general formula (I)

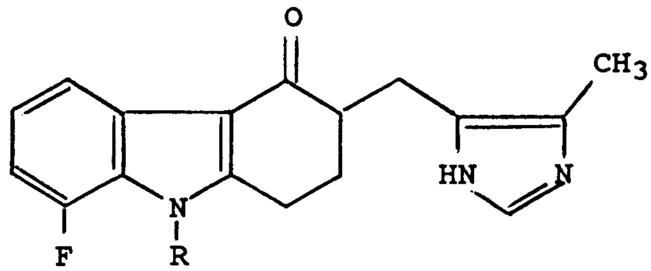

(I)

wherein R represents a hydrogen atom or a $C_{1-3}$alkyl group and physiologically acceptable salts and solvates thereof, which comprises:

(A) hydrogenating a compound of formula (II)

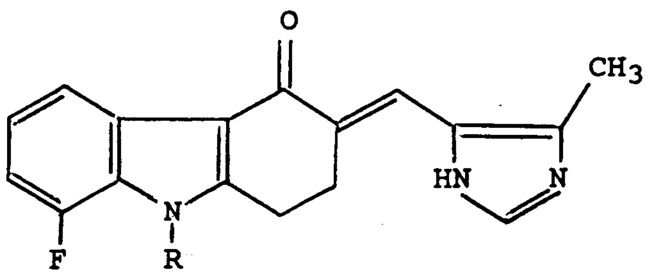

(II)

or a protected derivative thereof, followed where necessary by removal of any protecting group(s) present; or

(B) for the preparation of a compound of formula (I) in which R represents a $C_{1-3}$alkyl group, alkylating the compound of formula (I) in which R represents a hydrogen atom; or

(C) removing one or more protecting groups from a protected form of a compound of formula (I);

and when the compound of formula (I) is obtained as a mixture of enantiomers, optionally resolving the mixture to obtain the desired enantiomer; and/or

where the compound of formula (I) is in the form of a free base, optionally converting the free base into a salt.

**2.** A process as claimed in claim 1 for the preparation of the compound: 8-fluoro-1,2,3,9-tetrahydro-9-methyl-3-[(5-methyl-1H-imidazol-4-yl)methyl]-4H-carbazol-4-one or a physiologically acceptable salt or solvate thereof.

**3.** A process as claimed in claim 1 for the preparation of the compound: 8-fluoro-1,2,3,9-tetrahydro-3-[(5-methyl-1H-imidazol-4-yl)methyl]-4H-carbazol-4-one or a physiologically acceptable salt or solvate thereof.

**Patentansprüche**
**Patentansprüche für folgende Vertragsstaaten : AT, BE, CH, DE, FR, GB, IT, LI, LU, NL, SE**

1. Verbindungen der allgemeinen Formel (I):

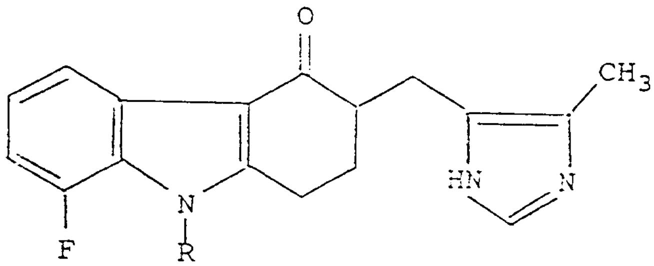

(I)

worin R ein Wasserstoffatom oder eine $C_{1-3}$-Alkylgruppe bedeutet, und die physiologisch annehmbaren Salze und Solvate davon.

2. Verbindung nach Anspruch 1, nämlich 8-Fluor-1,2,3,9-tetrahydro-9-methyl-3-[(5-methyl-1H-imidazol-4-yl)methyl]-4H-carbazol-4-on, und die physiologisch annehmbaren Salze und Solvate davon.

3. Verbindung nach Anspruch 1, nämlich 8-Fluor-1,2,3,9-tetrahydro-3-[(5-methyl-1H-imidazol-4-yl)methyl]-4H-carbazol-4-on, und die physiologisch annehmbaren Salze und Solvate davon.

4. Verfahren zur Herstellung von Verbindungen nach irgendeinem der Ansprüche 1 bis 3 und der physiologisch annehmbaren Salze und Solvate davon, dadurch **gekennzeichnet,** daß
(A) eine Verbindung der Formel (II):

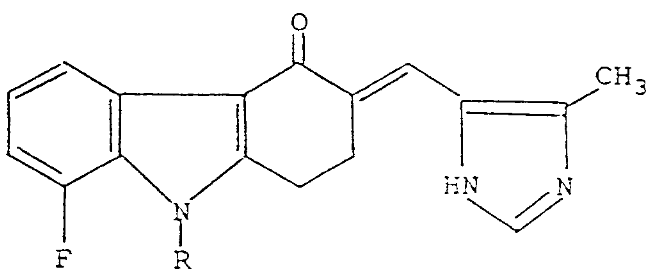

(II)

oder ein geschütztes Derivat davon hydriert wird, gefolgt, sofern erforderlich, von der Entfernung von irgendeiner vor
handenen Schutzgruppe bzw. vorhandenen Schutzgruppen; oder (B) für die Herstellung einer Verbindung der Formel (I), worin R eine $C_{1-3}$-Alkylgruppe bedeutet, die Verbindung der Formel (I), worin R ein Wasserstoffatom bedeutet, alkyliert wird; oder
(C) eine oder mehrere Schutzgruppe(n) aus einer geschützten Form der Verbindung der Formel (I) entfernt wird bzw. werden;
und wenn die Verbindung der Formel (I) als Gemisch der Enantiomeren erhalten wird, gegebenenfalls das Gemisch unter Bildung des gewünschten Enantiomeren gespalten wird; und/oder wenn die Verbindung der Formel (I) in Form einer freien
Base vorliegt, gegebenenfalls die freie Base in ihr Salz überführt wird.

5. Pharmazeutische Zusammensetzung, dadurch **gekennzeichnet,** daß es eine Verbindung der allgemeinen Formel (I), wie in einem der Ansprüche 1 bis 3 definiert, oder ein physiologisch annehmbares Salz oder Solvat davon zusammen mit mindestens einem physiologisch annehmbaren Träger oder Verdünnungsstoff enthält.

6. Verbindung der allgemeinen Formel (I) nach irgendeinem der Ansprüche 1 bis 3 oder ein physiologisch annehmbares Salz oder Solvat davon für die Verwendung als aktives therapeutisches Mittel.

**Patentansprüche für folgende Vertragsstaaten : ES, GR**

1. Verfahren zur Herstellung von Verbindungen der allgemeinen Formel (I):

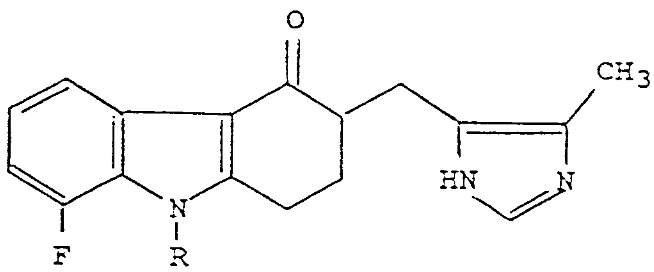

(I)

worin R ein Wasserstoffatom oder eine $C_{1-3}$-Alkylgruppe bedeutet, und der physiologisch annehmbaren Salze und Solvate davon, dadurch **gekennzeichnet,** daß
(A) eine Verbindung der Formel (II):

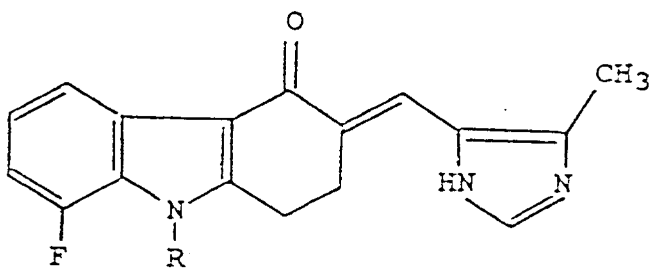

(II)

oder ein geschütztes Derivat davon hydriert wird, gefolgt, sofern erforderlich, von der Entfernung von irgendeiner vorhandenen Schutzgruppe bzw. vorhandenen Schutzgruppen; oder
(B) für die Herstellung einer Verbindung der Formel (I), worin R eine $C_{1-3}$-Alkylgruppe bedeutet, die Verbindung der Formel (I), worin R ein Wasserstoffatom bedeutet, alkyliert wird; oder
(C) eine oder mehrere Schutzgruppe(n) aus einer geschützten Form der Verbindung der Formel (I) entfernt wird bzw. werden;
und wenn die Verbindung der Formel (I) als Gemisch der Enantiomeren erhalten wird, gegebenenfalls das Gemisch unter Bildung des gewünschten Enantiomeren gespalten wird; und/oder
wenn die Verbindung der Formel (I) in Form einer freien Base vorliegt, gegebenenfalls die freie Base in ihr Salz überfuhrt wird.

2. Verfahren nach Anspruch 1, dadurch **gekennzeichnet,** daß 8-Fluor-1,2,3,9-tetrahydro-9-methyl-3-[(5-methyl-1H-imidazol-4yl)methyl]-4H-carbazol-4-on oder ein physiologisch annehmbares Salz oder Solvat davon hergestellt wird.

3. Verfahren nach Anspruch 1, dadurch **gekennzeichnet,** daß 8-Fluor-1,2,3,9-tetrahydro-3-[(5-methyl-1H-imidazol-4-yl)methyl]-4H-carbazol-4-on oder ein physiologisch annehmbares Salz oder Solvat davon hergestellt wird.

**Revendications**

**Revendications pour les Etats contractants suivants : AT, BE, CH, DE, FR, GB, IT, LI, LU, NL, SE**

**1.** Composés de la formule générale (I)

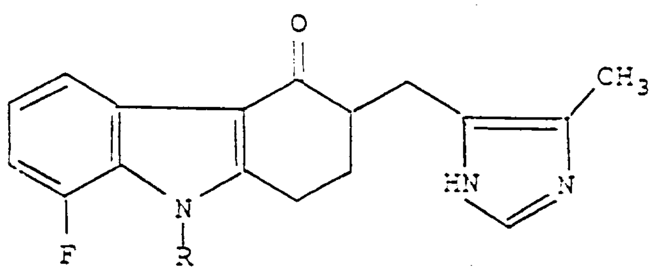

(I)

dans laquelle R représente un atome d'hydrogène ou un groupe alkyle en $C_1$ à $C_3$ ainsi que leurs sels et leurs produits de solvatation, physiologiquement acceptables.

**2.** Un composé tel que revendiqué dans la revendication 1, caractérisé en ce que c'est la 8-fluoro-1,2,3,9-tétrahydro-9-méthyl-3-[(5-méthyl-1H-imidazol-4-yl)méthyl]-4H-carbazol-4-one ainsi que ses sels et ses produits de solvatation, physiologiquement acceptables.

**3.** Un composé tel que revendiqué dans la revendication 1, caractérisé en ce que c'est la 8-fluoro-1,2,3,9-tétrahydro-3-[(5-méthyl-1H-imidazol-4-yl)méthyl]-4H-carbazol-4-one ainsi que ses sels et ses produits de solvatation physiologiquement acceptables.

**4.** Un procédé pour la préparation de composés tel que revendiqué dans l'une quelconque des revendications 1 à 3 ainsi que de leurs sels et de leurs produits de solvatation physiologiquement acceptables, selon lequel:

(A) on hydrogène un composé de formule (II)

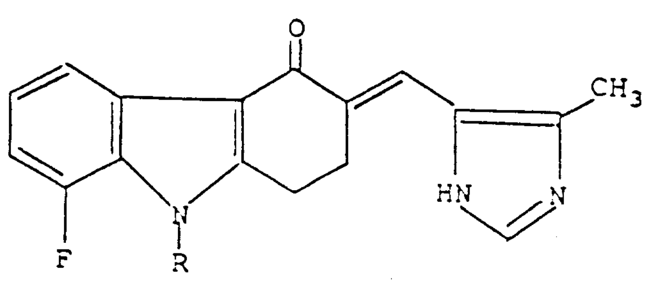

(II)

ou un de ses dérivés protégés, suivi, si nécessaire, par une élimination d'un (de) groupe(s) protecteur(s) quelconque(s) présent(s) ; ou

(B) pour la préparation d'un composé de formule (I) dans lequel R représente un groupe alkyle en $C_1$ à $C_3$, en alkylant le composé de formule (I) dans lequel R représente un atome d'hydrogène ; ou

(C) on élimine un ou plus d'un groupe protecteur à partir d'une forme protégée d'un composé de formule (I);

et lorsque le composé de formule (I) est obtenu sous la forme d'un mélange d'énantiomères, on résoud, le cas échéant, le mélange afin d'obtenir l'énantiomère désiré; et/ou

si le composé de formule (I) est sous la forme d'une base libre, on convertit, le cas échéant, la base libre en un sel.

**5.** Une composition pharmaceutique comportant un composé de formule générale (I) telle que définie dans l'une quelconque des revendications 1 à 3 ou un de ses sels ou de ses produits de solvatation, physiologiquement acceptables, conjointement avec au moins un support ou un excipient physiologiquement acceptable.

**6.** Un composé de la formule générale (I) tel que défini dans l'une quelconque des revendications 1 à 3 ou un de ses sels ou bien un de ses produits de solvatation, physiologiquement acceptables, destiné à être utilisé comme agent thérapeutique actif.

**Revendications pour les Etats contractants suivants : ES, GR**

**1.** Un procédé pour la préparation de composés de la formule générale (I)

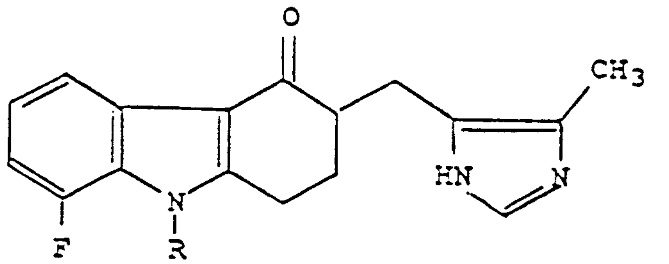

(I)

dans laquelle R représente un atome d'hydrogène ou un groupe alkyle en $C_1$ à $C_3$ ainsi que leurs sels et leurs produits de solvatation, physiologiquement acceptables, procédé selon lequel :

(A) on hydrogène un composé de formule (II)

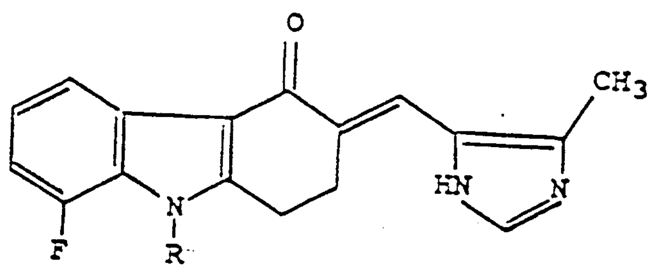

(II)

ou un de ses dérivés protégés, suivi, si nécessaire, par une élimination d'un (de) groupe(s) protecteur(s) quelconque(s) présent(s) ; ou

(B) pour la préparation d'un composé de formule (I) dans lequel R représente un groupe alkyle en $C_1$ à $C_3$, en alkylant le composé de formule (I) dans lequel R représente un atome d'hydrogène ; ou

(C) on élimine un ou plus d'un groupe protecteur à partir d'une forme protégée d'un composé de formule (I);

et lorsque le composé de formule (I) est obtenu sous la forme d'un mélange d'énantiomères, on résoud, le cas échéant, le mélange afin d'obtenir l'énantiomère désiré; et/ou

si le composé de formule (I) est sous la forme d'une base libre, on convertit, le cas échéant, la base libre en un sel.

**2.** Un procédé tel que revendiqué dans la revendication 1 pour la préparation du composé : 8-fluoro-1,2,3,9-tétrahydro-9-méthyl-3-[(5-méthyl-1H-imidazol-4-yl)méthyl]-4H-carbazol-4-one ainsi que ses sels et ses produits de solvatation, physiologiquement acceptables.

**3.** Un procédé tel que revendiqué dans la revendication 1 pour la préparation du composé : 8-fluoro-1,2,3,9-tétrahydro-3-[(5-méthyl-1H-imidazol-4-yl)méthyl]-4H-carbazol-4-one ainsi que ses sels et ses produits de solvatation physiologiquement acceptables.